# EUROPÄISCHE PATENTSCHRIFT

(11) **EP 1 824 528 B1**
(45) Veröffentlichungstag und Bekanntmachung des Hinweises auf die Patenterteilung: **21.01.2015**
(21) Anmeldenummer: 05812909.9
(22) Anmeldetag: 07.12.2005
(51) Int. Cl.: A61L 27/30, A61L 29/10, A61L 31/08

(54) **Implantat mit modifizierter Oberfläche**
Implant with modified surface
Implant avec surface modifiée

(30) Priorität: 07.12.2004 AT 20632004
(43) Veröffentlichungstag der Anmeldung: 29.08.2007
(73) Patentinhaber: DiaCoating GmbH, 6020 Innsbruck (AT)
(72) Erfinder: STEINMÜLLER-NETHL, Doris, A-6074 Rinn/Aldrans (AT); STEINMÜLLER, Detlef, A-6074 Rinn/Aldrans (AT); KLOSS, Frank, Rudolf, A-6020 Innsbruck (AT); GASSNER, Robert, A-6020 Innsbruck (AT); BONN, Günther, A-6170 Zirl (AT); HUCK, Christian, Wolfgang, A-6020 Innsbruck (AT); NAJAM-UL-HAQ, Muhammed, A-6020 Innsbruck (AT); RAINER, Matthias, A-6094 Grinzens (AT); STECHER, Günther, A-6091 Götzens (AT)
(74) Vertreter: Sonn & Partner Patentanwälte
(86) Internationale Anmeldenummer: PCT/AT2005/000495
(87) Internationale Veröffentlichungsnummer: WO 2006/060836

(56) Entgegenhaltungen:
- WO-A-02/080996
- WO-A-2004/006977
- WO-A1-01/41825
- DE-U1-202004 009 061
- HAUERT R: "A review of modified DLC coatings for biological applications" DIAMOND AND RELATED MATERIALS, ELSEVIER SCIENCE PUBLISHERS, AMSTERDAM, NL, Bd. 12, Nr. 3-7, März 2003 (2003-03), Seiten 583-589, XP004423721 ISSN: 0925-9635
- LASSETER T L ET AL: "Covalently modified silicon and diamond surfaces: Resistance to nonspecific protein adsorption and optimization for biosensing" JOURNAL OF THE AMERICAN CHEMICAL SOCIETY 25 AUG 2004 UNITED STATES, Bd. 126, Nr. 33, 25. August 2004 (2004-08-25), Seiten 10220-10221, XP002368733 ISSN: 0002-7863

## Beschreibung

Die vorliegende Erfindung betrifft Implantate.

In der biomedizinischen Wissenschaft ist das Gebiet der Gewebetechnologie, des sogenannten "Tissue Engineerings" oder "Cell-Engineerings", eine neue attraktive, hoffnungsvolle Methode, die unterschiedliche Ziele hat:
1.Zellen sollen kultiviert und vermehrt werden, um sie ggf. einem Patienten einzusetzen;
2.Gewebe soll *in vitro* kultiviert werden, um Untersuchungen und Analysen vornehmen zu können;
3.Wachstum und Organfunktionen sollen durch den Einfluss von Biomolekülen, Proteinen *in vitro* und *in vivo* beeinflusst werden.

Eine besondere Rolle spielt dabei die Anwendung von unterschiedlichsten Proteinen oder Biomolekülen, die das Zellwachstum *in vitro* und *in vivo* beeinflussen und modulieren sollen. Diese Proteine können endogenen (bilden sich im Körper selbst) oder exogenen (durch äußere Bedingungen ausgelöst) Ursprungs sein.

Das Einbringen dieser Proteine bzw. Biomoleküle in die Zellkultur oder in den Organismus kann über verschiedene Wege geschehen. In der Zellkultur werden solche Proteine in das Nährmedium gegeben und dadurch den in der Kultur befindlichen Zellen präsentiert. Dies startet einen nicht direkt beeinflussbaren Weg. Dieses Vorgehen entspricht *in vivo* einer systemischen Gabe eines Medikamentes oder eines Proteins. Beim Tissue-Engineering ist mit Ausnahme einer Medikamentengabe eine solche systemische Gabe in der Regel nicht erwünscht, da meist lokale, organspezifische Wirkungen erzielt werden sollen. Daher werden in der Literatur unterschiedliche Methoden angegeben, um Proteine an ihren Wirkort zu transportieren.

Die einfachste Methode ist die lokale Applikation von Proteinen, Biomolekülen oder Substraten an den gewünschten Wirkort. Dies kann zum Beispiel geschehen, indem diese Stoffe in einer Flüssigkeit suspendiert werden, die dann auf einen Träger aufgetrocknet werden, der schließlich in den Organismus eingebracht werden soll (AU 688406; WO 2004/024199). Durch die vorhandene, lokale Durchblutung und die Diffusionsvorgänge kann durch diese Anwendungstechnik eine systemische Wirkung nicht verhindert werden. Ein weiterer Nachteil besteht darin, dass bei der lokalen Applikation die Wirkung aufgrund dieses "single-shot" auf einen kurzen Zeitraum beschränkt ist und hohe Konzentrationen notwendig sind. Daher wurden Systeme entwickelt, die ein Einbringen der Proteine in den Organismus und gleichzeitig eine verzögerte Freisetzung der Proteine ermöglichen. Eine Methode stellt der Einsatz sogenannter "slow releasing systems" oder "biomimetic coatings" dar (WO 2004/024201; US 6,129,928; CN 1393218). Dabei können Proteine oder ähnliche Wirksubstanzen in ein resorbierbares bzw. degradierbares Material eingebettet und in den Organismus eingebracht werden. Dort werden dann die Proteine durch das Auflösen der Trägersubstanz nach und nach freigesetzt. Somit entsteht eine verzögerte Freisetzung der angewandten Substanzen, ein "single-Shot" Effekt kann vermieden werden, eine systemische Wirkung kann dagegen nicht vermieden werden.

Eine alternative Technik stellt der Transfer von Genabschnitten dar. Dadurch sollen ortsständige Zellen angeregt werden ein bestimmtes Protein auszuschütten oder sich zu differenzieren. Der Einsatz von Vektoren, darunter vor allem Adenoviren, hat sich dabei als besonders effizient erwiesen. Man bezeichnet die Viren als Vektoren, da sie Übermittler des entsprechenden Genabschnittes sind. Voraussetzung für die Nutzung der Viren zur Genübertragung ist es, sowohl die Aktivierung der viralen Promotoren als auch die Progression der lytischen Infektion, also die Zerstörung der Zielzellen zu unterdrücken. Trotzdem soll die direkte Infektion des Zielgewebes möglich bleiben und damit der Gentransfer ermöglicht werden. Diese Bedingungen können zum Beispiel im Adenovirus durch Deletion von bestimmten vireneigenen Genabschnitten erfüllt werden. Trotz der Deletion von für den Organismus schädlichen Genabschnitten werden virale Genprodukte in Zellen, welche mit Adenovektoren infiziert wurden, gefunden. Durch Anwendung von Liposomen können ebenfalls Vektoren in Zellen eingebracht werden, eine Infektiosität ist nicht gegeben (US 5,755,788). Liposome können an Oberflächen gebunden werden, auf diese Weise kann eine Aktivierung des umgebenden Gewebes erzielt werden (Thorwarth M et al., Mund Kiefer Gesichtschir. (2004); 8:250-255).

Den entscheidenden Vorteil der Nutzung dieser Therapieform stellt die Tatsache dar, dass eine Expression des transferierten Gens über einen längeren Zeitraum als bei herkömmlichen Trägermaterialien möglich ist. Nach sehr hoher initialer Expression fällt die Produktion der transferierten Gene meist erst nach ca. 4 Wochen ab. Diese Methode wird bereits in therapeutischen Ansätzen zur Mukoviszidosebehandlung genutzt.

Als nachteilig bei all den beschriebenen Methoden wirkt sich die Tatsache aus, dass durch die Applikation bzw. durch die Freisetzung der eingebrachten Substanzen eine systemische Wirkung nicht vermieden werden kann. Des Weiteren sind Dosen oberhalb der physiologischen Bedürfnisse notwendig, um den Abtransport, beispielsweise von einem Grundkörper weg, der als Implantat, chirurgisches Instrument oder Zellwachstumsbehälter verwendet wird, zu kompensieren.

Derartige "drug release" Systeme werden in der Implantologie eingesetzt. So wird beispielsweise in der DE 10216971 ein medizinisches Implantat offenbart, welches eine biokompatible Oberfläche bestehend aus einer kohlenstoffhaltigen Schicht und einem "drug release" System offenbart. Auch hierbei reduziert sich in kurzer Zeit die an der Oberfläche abgegebene Wirkstoffkonzentration bis auf ein Niveau, bei dem keine oder eine nicht ausreichende Wirkung im Körper mehr ausgelöst werden kann.

In der WO 02/080996 A1 wird die Beschichtung medizinischer Implantate mit einer diamant-ähnlichen Kohlenstoffbeschichtung beschrieben. Diese Beschichtung wird zusätzlich mit einer biodegradierbaren Beschichtung versehen, in der medizinisch relevante Substanzen eingebracht sind. Diese Substanzen sollen kontrolliert freigesetzt werden.

In der DE 101 07 795 A1 ist unter anderem ein Verfahren zur Verringerung von Folgekomplikationen bei der Implantation von Gefäßstützen beschrieben. Um derartige Komplikationen zu reduzieren, werden an den Implantaten Substanzen, vorzugsweise 17β-Östradiol, vorgesehen, welche das Wachstum von glatter Muskulatur verhindern und zur selben Zeit die Ausbildung des Endothels fördern. Über den Zeitraum der Einheilung soll es zu einer Freisetzung des Wirkstoffs 17β-Östradiol kommen.

Die US 2004/0198049 betrifft die Verwendung von "diamondoid" aufweisenden Materialien in der Mikroelektronik.

In der WO 2003/035924 wird ein Substrat geoffenbart, welches mit einer Beschichtung umfassend amorphen Kohlenstoff versehen ist, an welcher Metallionen immobilisiert sind, die eine antimikrobielle Wirkung aufweisen.

Die US 2003/0199741 betrifft eine medizinische Vorrichtung, welche mit Diamanten, DLC, Borosilikat, Karbiden und Nitriden versehen sind. Dabei ist auch ein Katheter vorgesehen, der eine Diamant- oder DLC-Beschichtung aufweist.

Die WO 1998/02100 betrifft Implantate, welche mit einem thromboresistenten Material versehen sind, wobei Beschichtungen, die DLC umfassen, als besonders vorteilhaft angesehen werden. Auf die DLC-Beschichtung können gemäß dieser Schrift antithrombogene oder thrombogene Wirkstoffe entweder direkt oder über eine weitere Beschichtung, wie beispielsweise bioabbaubare Matrizes, aufgebracht werden.

In der DE 101 52 055 ist ein Verfahren zur Abscheidung mechanisch und thermodynamisch stabiler amorpher Kohlenstoffschichten mit Hilfe eines Niederdruckplasma-Abscheideverfahrens geoffenbart. Unter anderem wird auch die Möglichkeit der Verwendung von amorphen Kohlenstoffschichten, auf die ein pharmazeutischer Wirkstoff aufgebracht ist, erwähnt.

Die DE 20 2004 000061 U1 betrifft medizinische Implantate mit einer kohlenstoffhaltigen DLC Schicht, welche durch Schaffung von Porosität und Funktionalisierung aktiviert wird.

Die WO 01/41825A1 betrifft medizinische Geräte zur Zelladhäsion, welche flexible Beschichtungen wie DLC aufweisen.

Kohlenstoffhaltige Schichten werden häufig zur Beschichtung von Implantaten herangezogen, da derartige Schichten eine hohe Biokompatibilität aufweisen und somit die Abstoßungsreaktionen derart stark reduzieren, dass zum Teil keine derartigen Reaktionen mehr hervorgerufen werden. Ferner erwiesen sich kohlenstoffhaltige Schichten auch als robust und eine geringe Reibung aufweisend (US 6,447,295; EP 0 302 717)

Somit ist es eine Aufgabe der vorliegenden Erfindung biokompatible Grundkörper, insbesondere Implantate zur Verfügung zu stellen, die es ermöglichen Wachstumsfaktoren, welche im Körper eines Menschen bzw. eines Tieres eine Reaktion bewirken, über einen langen Zeitraum in einer vorbestimmten Konzentration an einer vordefinierten Stelle der Umgebung auszusetzen, um dadurch beispielsweise das Wachstum von Zellen, insbesondere Körperzellen, oder Ossifikation nach Frakturen bzw. Knochenbrüchen zu fördern. Eine weitere Aufgabe der vorliegenden Erfindung ist es medizinische Mittel bereit zu stellen, die in einem Körper eines Säugetiers eine hohe Akzeptanz aufweisen und somit keine bzw. sehr geringe negative Körperreaktionen (z.B. Abstoßungsreaktionen) hervorrufen, wobei beispielsweise auch eine Steigerung der Osseointegration induziert werden kann.

Daher betrifft die vorliegende Erfindung ein Implantat umfassend ein zumindest teilweise mit einer kohlenstoffhaltigen Schicht überzogenes Substrat, dadurch gekennzeichnet, dass die kohlenstoffhaltige Schicht einen Diamantkristallanteil von mindestens 80% aufweist und zumindest teilweise mit Wachstumsfaktoren funktionalisiert ist, die direkt oder über mindestens einen Linker oder funktionelle Gruppe an die kohlenstoffhaltige Schicht gebunden sind. Die vorliegende Erfindung wird in den Ansprüchen definiert. Die weitere Beschreibung betrifft nun im Hinblick auf die Anspruchsgegenstände die vorliegende Erfindung.

Die zu beschichtenden Implantate werden in der Folge auch als Grundkörper bezeichnet.

Die Kohlenstoffatome der Beschichtung ermöglichen eine kovalente Bindung von Molekülen, wie z.B. Proteinen oder sonstigen Biomolekülen, oder von Linkern, an denen Biomoleküle kovalent oder nicht-kovalent gebunden werden können, an die kohlenstoffhaltige Schicht und somit an das Implantat (vgl. AT-A-589/2004). Die kohlenstoffhaltige Schicht muss dabei derart auf einen Grundkörper aufgebracht werden, dass dennoch die Mikrotextur und Porosität der Grundkörperoberfläche nicht gestört wird. Dies ist für die Osseointegration, also das knöcherne Einheilen solcher Grundkörper als Implantate von entscheidender Bedeutung, da in zahlreichen Untersuchungen gezeigt werden konnte, dass gerade die mikrostrukturierte Oberfläche ein Einwandern von Osteozyten an die Oberfläche erlaubt (Buser D et al., J Dent Res (2004) 83: 529-533), und erst dadurch eine Osseointegration gelingt. Beispielsweise wird in der AT 399726 B ein Verfahren beschrieben, welches es ermöglicht nanokristalline, reine Diamantschichten, auf Oberflächen aufzubringen, um den Anforderungen an Grundkörper gerecht zu werden. Da die Kohlenstoffatome als Bindungsstelle für Moleküle fungieren, besteht nach der Beschichtung die Oberfläche der Grundkörper zumindest teilweise aus Kohlenstoff. Dadurch stehen zahlreiche Bindungsstellen zur Verfügung, was es erlaubt, nicht nur ein Molekül, sondern eine Vielzahl von unterschiedlichen Molekülen an die Oberfläche zu binden. Dadurch kann zum Beispiel die Konzentration der gewünschten Moleküle durch Kombination mit Plazebomolekülen (Moleküle ohne biologische Wirkung; kompetitive Bindung der Plazebomoleküle und der biologisch wirksamen Moleküle) reduziert und den örtlichen, physiologischen Bedürfnissen angepasst werden.

Das "Substrat" selbst, als formgebender Bestandteil des Grundkörpers, kann aus beliebigen Materialien bestehen, die sich als Träger für kohlenstoffhaltige Schichten und für Grundkörper eignen, wobei die Substrate sowohl elektrisch leitfähig als auch elektrisch nicht leitfähig sein können.

"Funktionalisiert" im Sinne der vorliegenden Erfindung bedeutet, dass ein Wachstumsfaktor an die Oberfläche eines Grundkörpers derart gebunden wird, dass eine Abdiffusion des Wachstumsfaktors im Wesentlichen nicht oder nur geringfügig möglich ist und damit eine systemische Wirkung vermieden werden kann. Eine Diamantschichtoberfläche beispielsweise eignet sich ideal zur Anbindung von Proteinen (Nature Materials 3, 736 - 742 (01 Oct 2004)). Die Kohlenstoffatome können durch chemische und physikalische Methoden so aufgeschlüsselt werden, dass funktionelle Gruppen gebunden werden können. Durch die chemische Bindung wird eine lokale Wirkung sichergestellt. Die biologische Aktivität der Wachstumsfaktoren wird durch die kovalente Bindung an die kohlenstoffhaltige Oberfläche nicht beeinflusst und kann sich so lokal entfalten (Nature Materials 3, 736 - 742 (01 Oct 2004)). Neben kovalenten Bindungen können Wachstumsfaktoren auch durch nicht-kovalente Bindungen an die kohlenstoffhaltige Schicht gebunden werden, sofern diese Bindung derart stark ist, dass ein Abdiffundieren unter physiologischen Bedingungen in einem eingeschränkten Bereich bzw. nicht möglich ist (vgl. Biotin-Streptavidin-Bindung).

Erfindungsgemäß ist die auf der Oberfläche des Substrats befindliche kohlenstoffhaltige Schicht chemisch-physikalisch modifiziert, so dass erfindungsgemäße Wachstumsfaktoren daran gebunden werden können. Durch eine chemische Modifikation kann beispielsweise die Oberfläche derart verändert werden, dass daran Wachstumsfaktoren spezifisch oder selektiv gebunden werden können. Die Bindung der Wachstumsfaktoren kann kovalent oder über Affinitäts- bzw. Bioaffinitätschromatographie unter Verwendung von beispielsweise Aminosäurensequenzen an die chemisch und/oder physikalisch modifizierte kohlenstoffhaltige Schicht erfolgen. Das Vorhandensein von biologisch aktiven Wachstumsfaktoren auf einem erfindungsgemäßen Grundkörper erlaubt es, wenn mit biologischen Systemen (Zellkulturen, Gewebekulturen, Tieren, Menschen usw.) in Kontakt gebracht, diese zu beeinflussen.

Das Funktionalisieren der kohlenstoffhaltigen Schicht, vorzugsweise der Diamantschicht, kann direkt an die dangling bonds des Kohlenstoffs oder über einen Linker (erfindungsgemäß wird unter einem "Linker" eine chemische Verbindung mit einer funktionellen Gruppe verstanden, die entweder direkt an die reine und/oder chemisch-physikalisch modifizierte Diamantschicht oder an die reine und/oder chemisch-physikalisch modifizierte kohlenstoffhaltige Schicht oder an die funktionelle Gruppe eines weiteren "Linkers" andockt und bindet; der "Linker" kann selbst über eine Bindefunktionalität verfügen) erfolgen. Es können auch mehrere Substanzen an die kohlenstoffhaltige Oberfläche gebunden werden.

Im Rahmen der vorliegenden Erfindung wird mit "Bindefunktionalität" eine funktionelle Gruppe, die Wachstumsfaktoren, binden, vorzugsweise kovalent binden, kann, bezeichnet. Eine nicht-kovalente Bindung soll aber derart stark sein, dass unter physiologischen Bedingungen die an die kohlenstoffhaltige Schicht gebundenen Wachstumsfaktoren nicht oder nur in geringem Maße abdiffundieren.

Erfindungsgemäß versteht man unter einer "funktionellen Gruppe" jenen Teil eines Moleküls, der für die Bindung eines weiteren Moleküls verantwortlich ist. Diese funktionellen Gruppen (z.B. Aminogruppen, Hydroxylgruppen, Carboxylgruppen, Sulfhydrylgruppen) werden entsprechend der spezifisch zu bindenden Wachstumsfaktoren gewählt.

Kohlenstoffhaltige Schichten, insbesondere Diamantschichten, können mittels Galvanisierung auf ein Substrat aufgebracht werden.

Weitere Herstellungsverfahren lassen sich in drei wesentliche Kategorien einteilen: "hot-filament-Verfahren", "Plasmaverfahren" und "Hybrid-Verfahren". Weiters existieren noch Alternativtechnologien, die jedoch in der Anwendung derzeit wenig etabliert sind. Einen Überblick über verschiedene Technologien findet man zum Beispiel in "Diamond Films Handbook (edited by Jes Asmussen and D.K. Reinhard, Marcel Dekker, 2002, ISBN 0-8247-9577-6) oder in "Synthetic Diamond - Emerging CVD Science and Technology" (edited by K.E. Spear and J.P. Dismukes, The Electrochemical Society Series, John Wiley & Sons, ISBN 0-471-53589-3).

Das hot-filament Verfahren beruht auf der thermischen Anregung von kohlenstoffhaltigen Gasen im Niederdruck-Bereich. Dabei scheiden sich verschiedene Formen von kohlenstoffhaltigen Schichten auf einem Substrat ab. Durch die thermische Anregung eines zweiten Gases - meist Wasserstoff, der in atomaren Wasserstoff aufgespalten wird - werden anschließend die Komponenten weggeätzt, bei denen der Kohlenstoff in sp¹- oder sp²-Hybridisierung vorliegt. Bei geeigneter Parameterwahl ist somit die Aufbringung von kohlenstoffhaltigen Schichten mit sehr hohem kristallinen sp³-Hybrid-Anteil möglich. Eine Ausführungsform dieser Technologie ist in "Diamond and Related Materials" (P.K. Bachmann et al., 1991) und in der JP 2 092 895 beschrieben. Bei dem Plasma-Verfahren findet die Anregung der Gase durch eine Plasmaanregung in verschiedensten Ausführungsformen statt. Die Technologie beruht wieder auf dem oben beschriebenen Prinzip der Ablagerung verschiedenster Kohlenstoffmodifikationen, die ihrerseits wieder durch den angeregten atomaren Wasserstoff oder andere Hilfsgase, wie zum Beispiel Argon, geätzt werden, so dass in der Nettobilanz ein hoher Anteil an sp³-hybridisierten kristallinen Diamanten entsteht. Beispiele dieser Technologie finden sich in der JP 1 157 498 und in der EP 0 376 694.

Die Hybrid-Verfahren verwenden eine Kombination der beiden beschriebenen Technologien, d.h. die thermische Anregung durch Filamente wird durch verschiedenartige Plasmaanregungen unterstützt. Eine Ausführungsform ist in der US 4,504,519 beschrieben.

Ein weiteres bevorzugtes Herstellungsverfahren ist in der AT 399 726 B beschrieben. Hierbei handelt es sich um ein abgewandeltes hot-filament-Verfahren, bei dem die Gasanregung mit sehr hoher Effizienz betrieben werden kann. Mit diesem Verfahren können nicht nur DLC-Schichten und mikrokristalline Diamantschichten hergestellt werden, sondern auch sehr reine nanokristalline Diamantschichten, die sich in der hier beschriebenen Grundkörper-Beschichtung als besonders vorteilhaft erwiesen haben.

Vorzugsweise besteht die kohlenstoffhaltige Schicht aus polykristallinen, mikrokristallinen, nanokristallinen, ultrananokristallinen (John A. Carlisle and Orlando Auciello, Ultrananocrystalline Diamond- Properties and Applications in Biomedical Devices, The Electrochemical Society Interface, 12 (1), 28-31 (2003)) oder monokristallinen Diamantkristallen.

Im Gegensatz zu den im Stand der Technik genannten Oberflächen liegt der Vorteil des erfindungsgemäßen Grundkörpers in der hohen Biokompatibilität, der hohen chemischen Stabilität, in der Regenerierbarkeit und in der definierten chemischen und physikalischen Modifikation/Funktionalisierung der kohlenstoffhaltigen Oberfläche, insbesondere da die Oberfläche Diamanten umfasst.

Der Begriff "Biokompatibilität" bezieht sich auf den Grundkörper des erfindungsgemäßen Implantats und bedingt, dass er sowohl in reiner als auch in chemisch bzw. physikalisch modifizierter Form die Umgebung weder negativ beeinflusst noch zerstört.

Aus der Literatur ist bekannt, dass reiner Diamant biokompatible Eigenschaften aufweist (Wensha Yang et al., DNA-modified nanocrystalline diamond thin-films as stable, biologically active substrates; Nature Materials, November 24, 2002, 253-257). Durch entsprechende Vorbehandlung der Diamantschichten können Eigenschaften erreicht werden, die die Biokompatibilität gegenüber einzelnen Substanzen drastisch und vor allem dauerhaft erhöhen.

Durch die Vielzahl möglicher chemischer Modifikationen - speziell bei Diamantschichten, die ein sehr breites elektrochemisches Fenster für Anbindungen von Biomolekülen besitzen (Nature Materials 3, 736 - 742 (01 Oct 2004)) - können beispielsweise kontrollierte Beeinflussungen des Differenzierungsgrades von Zellen, der zelleigenen Expression von Proteinen, der Ausschüttung von chemotaktischen Substanzen, das heißt von Lockstoffen, aber auch die Beeinflussung von Zellabwehrmechanismen erfolgen. Die zusätzliche Möglichkeit, lokalisierte Bereiche (mit unterschiedlichen Eigenschaften) der kohlenstoffhaltigen Oberfläche (speziell einer nanokristallinen (auch poly-, mono- oder ultrananokristallinen) Diamantschicht) spezifisch zu funktionalisieren, erweitert den Anwendungsbereich für die Anbindung von unterschiedlichen Wachstumsfaktoren auf einer Grundkörper-O-berfläche.

Eine weitere Eigenschaft einer nanokristallinen Diamantoberfläche, nämlich die Widerstandsfähigkeit gegenüber Bakterien (Jakubowski W. et al., Nanocrystalline diamond surface is resitant to bacterial colonization, Diamond and Related materials, Volume 13, Issue 10, October 2004, 1761-1763) ist für die Anwendungen solcher Grundkörper von Vorteil.

Gemäß der Erfindung weist die kohlenstoffhaltige Schicht einen Diamantkristallanteil von mindestens 80%, vorzugsweise von mindestens 90%, von mindestens 95%, von mindestens 99%, insbesondere von mindestens 99,5%, auf.

Gemäß einer weiteren bevorzugten Ausführungsform weist die kohlenstoffhaltige Schicht Diamantkristalle mit einer Kristallitgröße von weniger als 500 nm, vorzugsweise von 5 bis 100 nm, insbesondere von 8 bis 30 nm, auf.

Erfindungsgemäß ist es günstig, wenn die kohlenstoffhaltige Schicht als Diamantschicht eine Kristallitgröße von weniger als 500 nm, vorzugsweise von weniger als 300 nm, insbesondere von weniger als 100 nm, aufweist. Diese Kristallitgrößen sind besonders vorteilhaft bei der Herstellung von speziellen Morphologien des Grundkörpers. Auch andere Kristallitgrößen sind erfindungsgemäß verwendbar.

Vorzugsweise weist die kohlenstoffhaltige Schicht eine Schichtdicke von 100 nm bis 50 µm, bevorzugt 100 nm bis 40 µm, insbesondere von 1 bis 20 µm, auf.

Erfindungsgemäß kann die kohlenstoffhaltige Schicht unterschiedlich dick, geschlossen oder nicht geschlossen ausgeführt sein, um jedenfalls optimale Ergebnisse bei der Funktionalisierung und/oder Beeinflussung bestimmten Zellverhaltens zu erzielen. Daher sind auch geringe Schichtdicken, bei denen die Schicht noch nicht geschlossen ist, durchaus möglich, um die Kosten zu reduzieren.

Gemäß einer bevorzugten Ausführungsform ist die kohlenstoffhaltige Schicht elektrisch isolierend oder elektrisch leitend, wobei die elektrische Leitfähigkeit aufgrund von Dotierung leitfähig ist.

Dabei wird das Volumsmaterial oder die Oberfläche des Diamanten mit den im Stand der Technik bekannten Elementen, wie beispielsweise Bor oder Brom oder Phosphor oder Stickstoff, dotiert. Beispiele für solche dotierten Diamantschichten finden sich in "Thin Film Diamond" (edited by A. Lettington and J.W. Steeds, III. Royal Society (GB), 1994, ISBN 0412496305). Elektrische Leitfähigkeit kann beispielsweise bei Operationen von Bedeutung sein, wenn zur Verödung oder dgl. elektrische Spannungen angelegt werden.

Gemäß einer weiteren bevorzugten Ausführungsform ist die kohlenstoffhaltige Schicht auf dem Grundkörper des Implantats aufgrund von adsorbierten Substanzen leitfähig. Durch die Adsorption von Wasserstoff wird beispielsweise die Oberfläche von Diamant leitfähig (O.A. Williams and R.B. Jackman, Surface conductivity on hydrogen terminated diamond, Semicond. Sci. Technol. 18, 34-40 (2003)). Eine Oberflächenleitfähigkeit kann durch Absättigung der sogenannten freien Bindungen (dangling bonds) durch Wasserstoff erreicht werden Eine solche wasserstoff-terminierte Diamantschicht-Oberfläche zeigt hydrophoben Charakter, während eine Terminierung der freien Bindungen durch Sauerstoff eine hydrophile Oberfläche verursacht. Eine solche sauerstoff-terminierte Oberfläche ist wiederum elektrisch nicht leitend.

Vorzugsweise umfasst das Substrat ein Material ausgewählt aus der Gruppe bestehend aus Metall, insbesondere Gold, Stahl, Molybdän und Titan, Metalllegierungen, insbesondere Stahllegierungen, Molybdänlegierungen und Titanlegierungen, Hartmetallen, Cermets, Metalloxiden, mineralischen Oxiden, Kohlenstoff, insbesondere Pyrokohlenstoff und Grafit, Halbleiter, Polymer, Kunststoff, insbesondere kohlefaser- bzw. glasfaserverstärkte Kunststoffe, Keramik (z.B. Si₂N₃, Al₂O₃, Zirkonoxid), Porzellan, Glas, Quarzglas, Kieselgel, Komposit-Materialien, Nanotubes, Nanowires, Nanopartikel, Fullerene, Siliziumverbindungen, insbesondere Silikon, Metallsilizide und Siliziumkarbid, Saphir, Multi-Matrix-Compounds (MMC), Glassy Carbon, Zellulose und Mischungen davon.

Zwischen dem Substrat und der kohlenstoffhaltigen Schicht kann mindestens eine Zwischenschicht aufgebracht sein.

Die Zwischenschicht umfasst vorzugsweise ein Material ausgewählt aus der Gruppe bestehend aus Metall, Metalllegierungen, Metalloxiden, Metallkarbiden, Siliziumverbindungen, insbesondere Metallsilizide, wie Kobaltsilizid, und Siliziumkarbid, mineralischen Oxiden, Grafit, Halbleiter, Polymer, Kunststoff, Keramik, Glas, Quarzglas, Kieselgel, Stahl, Stahllegierungen, Komposit-Materialien, Nanotubes, Nanowires, Nanopartikel, Fullerene, Pyrokohlenstoff, Glassy Carbon und Mischungen davon.

Eine Zwischenschicht bietet sich für Substrate an, die einen unterschiedlichen Wärmeausdehnungskoeffizienten zur kohlenstoffhaltigen Schicht, insbesondere Diamantschicht, aufweist. Durch eine solche Zwischenschicht können Schichtspannungen abgebaut und somit die Schichthaftung verbessert werden. Weiters ist es durch Zwischenschichten möglich, Substrate zu beschichten, die mit der Kohlenstoffschicht eine chemische Bindung eingehen würden. Ein Beispiel dafür sind Hartmetalle, bei denen durch eine geeignete Zwischenschicht die Kobalt-Bestandteile des Hartmetalls chemisch deaktiviert bzw. abgedeckt (passiviert) werden und somit die Bildung von Kobalt-Karbid entweder verhindert oder in für die Kohlenstoffschicht nicht aktive Bereiche verlegt werden. Es können mehrere Zwischenschichten vorgesehen werden, so dass ein Multilayer-System auf dem Substrat ausgebildet wird. Beispielsweise kann die mehrlagige Schicht eine Diamant-, eine DLC- und eine Polymer-Schicht umfassen.

Gemäß einer weiteren Ausführungsform weist die kohlenstoffhaltige Schicht hydrophile und/oder hydrophobe Bereiche auf.

Auf der Oberfläche der kohlenstoffhaltigen Schicht können Bereiche vorgesehen sein, die unterschiedliche chemische und/oder physikalische Eigenschaften aufweisen können, wie z.B. hydrophile, hydrophobe oder unterschiedlich funktionalisierte Bereiche. Diese Bereiche können durch bestimmte Technologien (Masken, Lithografie, Elektronenstrahllithografie etc.) strukturiert und durch anschließende Terminierung zum Beispiel in einem Sauerstoff- oder Wasserstoffplasma, nasschemisch, in Ozon-Atmosphäre oder in atomarer Wasserstoff-Atmosphäre kontrolliert hergestellt werden. Durch die entsprechende Oberflächenterminierung, zum Beispiel bei Diamantschichten an den sogenannten dangling bonds, kann durch Wasserstoff eine hydrophobe und durch Sauerstoff eine hydrophile Oberflächeneigenschaft erzeugt werden. Dabei kann die Oberfläche so gestaltet sein, dass die hydrophilen Bereiche, auf denen die Biomoleküle aufgetragen werden, von hydrophoben Bereichen begrenzt sind. Somit ist es möglich, dass spezielle - unterschiedliche - Biomoleküle bzw. Proteine lokalisiert zur Verfügung gestellt werden können, was bisher nicht möglich gewesen ist. Die Herstellung von hydrophilen bzw. hydrophoben Bereichen auf der Diamantoberfläche erfolgt gemäß den im Stand der Technik offenbarten Methoden (US 2002/045270 A1). Diese Strukturierung ist nicht nur auf die Eigenschaften der Hydrophobie bzw.

Hydrophilie beschränkt, sondern sie kann beliebige andere physikalische und/oder chemische Eigenschaften erfassen. Insbesondere kann natürlich auch die Funktionalisierung direkt strukturiert werden.

Vorzugsweise sind die hydrophilen und hydrophoben Bereiche der kohlenstoffhaltigen Oberfläche dabei strukturiert.

Gemäß einer bevorzugten Ausführungsform weist die chemisch und/oder physikalisch modifizierte kohlenstoffhaltige Schicht mindestens eine Bindefunktionalität ausgewählt aus der Gruppe bestehend aus polaren, apolaren, hydrophoben, hydrophilen, ionischen, affinen, spezifischen, metallkomplexierenden Gruppen und Mischungen davon, auf.

Erfindungsgemäß zählen zu "affinen" Gruppen all jene funktionellen Gruppen bzw. Moleküle, die gegenüber anderen chemischen Verbindungen und Gruppen eine Affinität aufweisen.

"Spezifische" funktionelle Gruppen umfassen alle chemischen Verbindungen, die andere chemische Verbindungen und Gruppen spezifisch zu binden vermögen. Beispielhaft erwähnt seien in diesem Zusammenhang Antikörper-Antigen-, Enzym-Substrat-, Enzym-Inhibitor- und Protein-Ligand-Verbindungen.

Vorzugsweise weist die kohlenstoffhaltige Schicht durch chemische Modifikation Wasserstoffatome, Halogene, Hydroxylgruppen, Carbonylgruppen, aromatische Ringsysteme, Schwefel, Schwefelderivate, Grignardverbindungen, Aminogruppen, Epoxide, Metalle oder Kohlenstoffketten an der Oberfläche auf.

Gemäß einer bevorzugten Ausführungsform weist die kohlenstoffhaltige Schicht mindestens eine Bindefunktionalität ausgewählt aus der Gruppe, bestehend aus Kohlenstoff-Doppelbindungen, Epoxiden, Halogenen, Aminogruppen, Hydroxygruppe, Säuregruppen, Säurechloriden, Cyanidgruppen, Aldehydgruppen, Sulfatgruppen, Sulfonatgruppen, Phosphatgruppen, Metall-komplexierenden Gruppen, Thiethern, Biotin, Thiolen und Mischungen davon, an der Oberfläche auf.

Vorzugsweise ist die kohlenstoffhaltige Schicht kovalent mit Wasserstoff (-H) (Toshiki Tsubota et al., Reactivity of the hydrogen atoms on diamond surface with various radical initiators in mild condition, Diamond and Related materials, 11 (7) 1360-1365 (2002)), Halogenen (-Cl, -Br, -I, -F), Hydroxylfunktion (-OH), Carbonylfunktion (=O), aromatischen Ringsystemen, Schwefel und Schwefelderivaten, Grignardverbindungen (-MgBr), Aminen (-NH₂), Epoxiden, Metallen (-Li) oder Kohlenstoffketten modifiziert. Die chemisch-physikalisch modifizierte kohlenstoffhaltige Schicht verfügt gegebenenfalls über Bindefunktionalitäten, wodurch das direkte kovalente Binden von Biomolekülen ermöglicht oder die Voraussetzung für eine weitere Funktionalisierung über einen Linker geschaffen wird.

Vorzugsweise ist die kohlenstoffhaltige Schicht mit mindestens einem Linker chemisch modifiziert, wobei der Linker mindestens eine Bindefunktionalität umfasst, welche bevorzugterweise ausgewählt aus den Gruppen, bestehend aus Kohlenstoff-Doppelbindungen, Epoxiden, Halogenen, Aminogruppen, Hydroxygruppe, Säuregruppen, Säurechloriden, Cyanidgruppen, Aldehydgruppen, Sulfatgruppen, Sulfonatgruppen, Phosphatgruppen, Metall-komplexierenden Gruppen, Thiethern, Biotin, Thiolen und Mischungen davon, ist.

Durch das direkte Aufbringen von funktionellen Gruppen auf die kohlenstoffhaltige Oberfläche ist es möglich die gewünschten Wachstumsfaktoren an den Grundkörper zu binden.

Der bzw. die Linker wird/werden dabei mit an sich im Stand der Technik bekannten Methoden (M.A. Fox and J.K. Whitesell, Organische Chemie, 1995, S. 255, 297-298, 335-338, 367-368, 406-408, 444-446, 493-496, 525-526, 550-551, 586-587, 879-884) an die chemisch-physikalisch modifizierte kohlenstoffhaltige Schicht, z.B. eine Diamantschicht, gebunden (beispielsweise wird eine Verbindung, enthaltend eine Kohlenstoff-Doppelbindung, durch photochemische Reaktionen an die Diamantschicht gebunden (Todd Strother et al., Photochemical Functionalisation of Diamond Films, Langmuir 18 (4): 968-971 (2002)).

Das Einbringen der Wachstumsfaktoren, gebunden an die kohlenstoffhaltige Oberfläche eines erfindungsgemäßen Grundkörpers, vorzugsweise eines mit mono-, mikro- oder nanokristalliner Diamantschicht beschichteten Substrats, erlaubt definitionsgemäß eine lokale Wirkung ohne Abdiffusion, womit eine systemische Wirkung verhindert werden kann.

Vorzugsweise sind am Grundkörper Proteine, insbesondere aus der TGF-β-Superfamilie, an die chemisch und/oder physikalisch modifizierte kohlenstoffhaltige Schicht gebunden.

Wachstumsfaktoren eignen sich gut zur Bindung an den Grundkörper eines erfindungsgemäßen Implantats, da es dadurch beispielsweise ermöglicht wird, in einem biologischen System das Wachstum eines den Grundkörper umgebenden Gewebes zu beeinflussen.

Gemäß einer bevorzugten Ausführungsform sind am Grundkörper Bone Morphogenetic Proteins (z.B. BMP-2) an der chemisch und/oder physikalisch modifizierten kohlenstoffhaltigen Schicht gebunden.

Ein Ziel dieser Erfindung stellt die Beschleunigung der Einheilzeit von Implantaten dar. Eine Möglichkeit der Verkürzung der Einheilzeit stellt die Nutzung von Wachstumsfaktoren in der Implantologie dar. Zu den wichtigsten osteogenen Wachstums- und Differenzierungsfaktoren zählen die zur TGF-gamma-Superfamilie gehörenden Bone morphogenetic proteins (BMP), von denen es mindestens 18 (BMP-2 bis 18) verschiedene gibt. BMP-2 und -7 sind derzeit die wichtigsten dieser Gruppe, die zur Induktion und Differenzierung von humanen Osteoblasten aus Knochenmark-Stromazellen führen. Zahlreiche tierexperimentelle Untersuchungen konnten diesen Effekt belegen. In der klinischen Forschung steht der Einsatz der Zytokine zur Regeneration von Knochendefekten im Vordergrund. Ein Ansatz liegt in der direkten Applikation von BMP's, in Kombination mit unterschiedlichen Trägermaterialien, auf einen knöchernen Defekt. Dabei konnte in tierexperimentellen Arbeiten eine signifikant verstärkte knöcherne Regeneration erzielt werden. Im Rahmen der Implantologie finden Zytokine vorwiegend experimentell Einsatz, da bislang eine systemische Wirkung durch die angewandten Methoden nicht verhindert werden kann. Vorwiegend zum Einsatz kommen das Einbringen der Proteine in den Implantatschacht, das Auftrocknen der Proteine auf das Implantat (AU 688406; WO 2004/024199) oder der Einsatz von "biomimetic coatings" (WO 2004/024201; US 6,129,928; CN 139318).

Die dargestellten Anwendungen gelten neben Implantaten gleichermaßen für im Knochen verankerte Prothesen und künstliche Gelenke, da hier ähnliche Anforderungen gestellt werden können. Auch hier kann das Einwachsverhalten beeinflusst werden.

Die Implantatoberflächen von zum Beispiel Teilen in der Traumatologie, die nicht mit dem Knochen zusammen wachsen sollen, besitzen keine strukturierte bzw. poröse Oberfläche, sondern sind glatt. Um diese Topografie zu erhalten, eignet sich eine Kohlenstoffschicht, im besonderen eine nanokristalline Diamantschicht, welche gezielt strukturiert und/oder funktionalisiert werden kann, um den gewünschten Einfluss vor Ort zu gewährleisten.

Erfindungsgemäß besteht ein Implantat" aus einem Substrat und einer kohlenstoffhaltigen Schicht. Auf diesem Implantat werden die Wachstumsfaktoren aufgebracht. Diese Implantate können sowohl bei Menschen als auch bei Tieren eingesetzt werden.

Wachstumsfaktoren können erfindungsgemäß kovalent an ein mit Kohlenstoffschichten, insbesondere mit nanokristallinen Diamantschichten, beschichtetes Implantat gebunden werden und damit eine lokale Wirkung, das heißt eine verbesserte und beschleunigte Osseointegration gerade im schwachen Knochenlager erzielt werden. Eine systemische Wirkung kann damit vermieden werden, womit ein routinemäßiger klinischer Einsatz möglich werden könnte.

Gemäß einer weiteren bevozugten Ausführungsform ist das Implantat ausgewählt aus der Gruppe bestehend aus Dentalimplantat, Knieimplantat, Hüftimplantat, Bolzen, Schrauben, Nagel, Herzklappen, Katheter, Stent, Platten, Schädelplatten, Schienen und Prothesen.

Die Erfindung wird anhand der folgenden Figuren und Beispiele näher erläutert, ohne jedoch auf diese beschränkt zu sein.

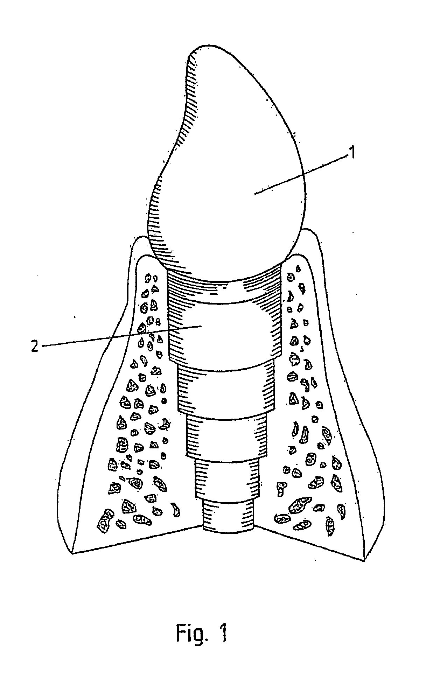

Fig. 1 zeigt einen künstlichen Zahn aus Keramik 1, welcher mittels eines Dentalimplantats aus einer Titanlegierung 2 in den Kiefer eingeschraubt wird.

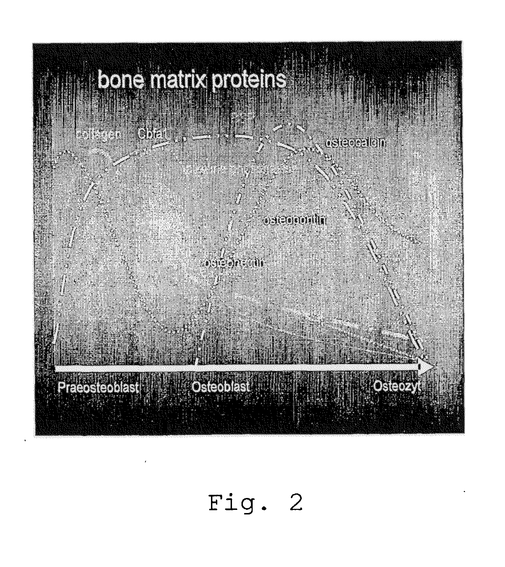

Fig. 2 ist ein schematische Darstellung der wichtigsten Knochenmatrixproteine in ihrem zeitlichen Auftreten bei der Differenzierung vom Präosteoblasten zur reifen Knochenzelle. Durch das unterschiedlich zeitliche Auftreten und die dadurch mögliche Zugabe kann die Differenzierung zur knochenbildenden Zelle in jeder Phase der Reifung beeinflusst werden.

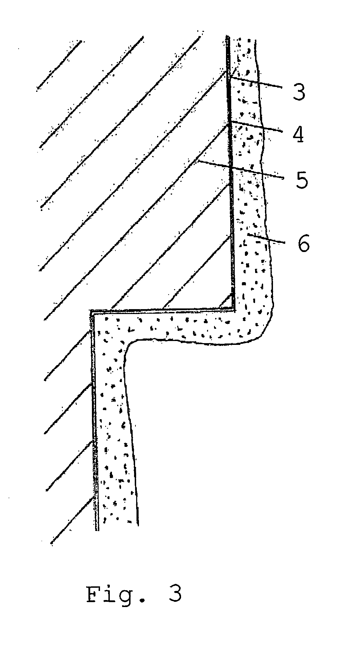

Fig. 3 zeigt eine nanokristalline, diamantbeschichtete 3 Grundkörperoberfläche 4, die chemisch durch ein Protein (BMP2) modifiziert wurde 5 und damit die Knochenbildung im Kiefer vor Ort beschleunigt 6.

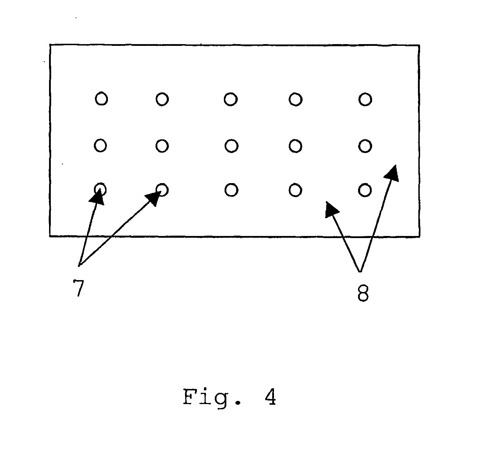

Fig. 4 zeigt eine strukturierte nanokristalline Diamantschicht auf einem Substrat (hydrophile Bereiche - lokalisiert im Kreis 7 und hydrophobe Bereiche in der Umgebung der Kreisbereiche 8) als Grundlage zur Anbindung unterschiedlicher Proteine, damit sind unter anderem auch Konzentrationsvariationen vor Ort möglich.

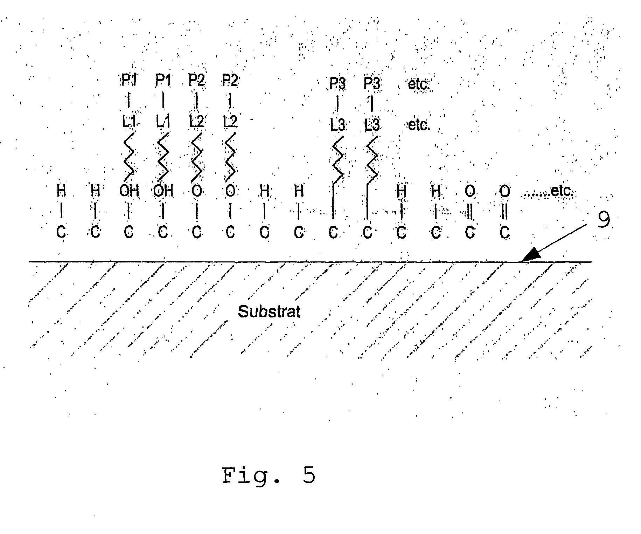

Fig. 5 zeigt ein Substrat, welches eine Diamantschicht 9 mit strukturierter Oberfläche aufweist, die lokalisiert eine Sauerstoff- oder Wasserstoff-Terminierung besitzt. Der entsprechende Linker/funktionelle Gruppe (L1, L2) stellt das Bindeglied für die Anbindung des gewünschten Proteins (P1 oder P2).

### BEISPIEL 1: Zahnimplantat

Bei der Rehabilitation des Kauapparates nach Zahnverlust, traumatischen oder tumorbedingten Defekten, sowie der Versorgung von Gesichtsdefekten nach Tumoroperationen oder angeborenen Anomalien durch Gesichtsprothesen, haben sich enorale, also im Kiefer befestigte (Fig. 1), und extraorale, also am Gesichtsschädel befestigte Implantate als Verankerungselemente bewährt. Ein entscheidender Faktor für die Erfolgssicherheit enossaler, d.h. im Knochen verankerter Implantate ist das Ausmaß des Knochenverbunds bzw. Einheilens, der sogenannten Osseointegration. Die Osseointegration hängt dabei entscheidend von der Beschaffenheit des ortsständigen Knochens ab. So ist die Erfolgssicherheit von Implantaten im vorbestrahlten Empfängerbett reduziert. Die verminderte Durchblutung in diesen Bereichen führt zu einem geringeren Regenerationspotenzial des Knochens nach chirurgischer Manipulation, so dass auch die Osseointegration von Implantaten gefährdet sein kann. Zur Verbesserung der Osseointegration wurden daher unterschiedliche Oberflächenbeschichtungen konzipiert (Buser D et al., J Dent Res (2004) 83: 529-533; Thorwarth M et al., Mund Kiefer Gesichtschir (2004) 8:250-255; Cochran DL et al., J Periodontol (1999) 70: 139-150).

### BEISPIEL 2: Modifikation eines Grundkörpers

Der Grundkörper wird in eine Illuminationskammer gegeben, die mit Stickstoff durchströmt wird. Die Abdeckung (Deckel) der Illuminationskammer besteht aus Quarzglas. Auf die H-terminierte kohlenstoffhaltige Schicht des Grundkörpers wird die Linkersubstanz, vorzugsweise geschütztes Trifluoracetamid mit langkettigem, ungesättigtem Amin, insbesondere 3-Aminopropen, aufgebracht und für 8 bis 12 Stunden mit UV-Licht illuminiert. Die Schutzgruppe des Trifluoracetamides wird durch eine methanolische-HCl Lösung bei 65° C entfernt. Anschließend wird am Amin des Linkers das Protein, vorzugsweise aus der TGF-β-Superfamilie, insbesondere das Bone Morphogenetic Protein (z.B. BMP-2), kovalent gebunden.

## Patentansprüche

1. Implantat umfassend ein zumindest teilweise mit einer kohlenstoffhaltigen Schicht überzogenes Substrat, **dadurch gekennzeichnet, dass** die kohlenstoffhaltige Schicht einen Diamantkristallanteil von mindestens 80% aufweist und zumindest teilweise mit Wachstumsfaktoren funktionalisiert ist, die direkt oder über mindestens einen Linker oder funktionelle Gruppe an die kohlenstoffhaltige Schicht gebunden sind.

2. Implantat nach Anspruch 1, **dadurch gekennzeichnet, dass** die kohlenstoffhaltige Schicht weiters ein Material ausgewählt aus der Gruppe bestehend aus Polymeren, amorphem Kohlenstoff, DLC (diamond-like-carbon), Grafit, Nanotubes, Nanowires, Nanopartikeln, Fullerenen, Pyrokohlenstoff, Glassy Carbon und Mischungen davon, umfasst.

3. Implantat nach Anspruch 1 oder 2, **dadurch gekennzeichnet, dass** die kohlenstoffhaltige Schicht polykristalline, mikrokristalline, nanokristalline, ultrananokristalline oder monokristalline Diamantkristalle umfasst.

4. Implantat nach einem der Ansprüche 1 bis 3, **dadurch gekennzeichnet, dass** die kohlenstoffhaltige Schicht Diamantkristalle mit einer Kristallitgröße von weniger als 500 nm aufweist.

5. Implantat nach Anspruch 4, **dadurch gekennzeichnet, dass** die kohlenstoffhaltige Schicht Diamantkristalle mit einer Kristallitgröße von 5 bis 100 nm aufweist.

6. Implantat nach Anspruch 5, **dadurch gekennzeichnet, dass** die kohlenstoffhaltige Schicht Diamantkristalle mit einer Kristallitgröße von 8 bis 30 nm aufweist.

7. Implantat nach einem der Ansprüche 1 bis 6, **dadurch gekennzeichnet, dass** die kohlenstoffhaltige Schicht eine Schichtdicke von 100 nm bis 50 µm aufweist.

8. Implantat nach Anspruch 7, **dadurch gekennzeichnet, dass** die kohlenstoffhaltige Schicht eine Schichtdicke von 100 nm bis 40 µm aufweist.

9. Implantat nach Anspruch 8, **dadurch gekennzeichnet, dass** die kohlenstoffhaltige Schicht eine Schichtdicke von 1 bis 20 µm aufweist.

10. Implantat nach einem der Ansprüche 1 bis 9, **dadurch gekennzeichnet, dass** die chemisch und/oder physikalisch modifizierte kohlenstoffhaltige Schicht mindestens eine Bindefunktionalität ausgewählt aus der Gruppe bestehend aus polaren, apolaren, hydrophoben, hydrophilen, ionischen, affinen, spezifischen, metallkomplexierenden Gruppen und Mischungen davon, aufweist.

11. Implantat nach einem der Ansprüche 1 bis 10, **dadurch gekennzeichnet, dass** die kohlenstoffhaltige Schicht durch chemische Modifikation Wasserstoffatome, Halogene, Hydroxylgruppen, Carbonylgruppen, aromatische Ringsysteme, Schwefel, Schwefelderivate, Grignardverbindungen, Aminogruppen, Epoxide, Metalle oder Kohlenstoffketten aufweist.

12. Implantat nach einem der Ansprüche 1 bis 10, **dadurch gekennzeichnet, dass** die kohlenstoffhaltige Schicht einen Diamantkristallanteil von mindestens 90% aufweist.

## Claims

1. Implant comprising a substrate which is at least partially coated with a carbon-containing layer, **characterized in that** the carbon-containing layer has a diamond-crystal proportion of at least 80% and is at least partially functionalized with growth factors which are bound to the carbon-containing layer directly or via at least one linker or functional group.

2. Implant according to claim 1, **characterized in that** the carbon-containing layer further comprises a material selected from the group consisting of polymers, amorphous carbon, DLC (diamond-like carbon), graphite, nanotubes, nanowires, nanoparticles, fullerenes, pyrocarbon, glassy carbon, and mixtures thereof.

3. Implant according to claim 1 or 2, **characterized in that** the carbon-containing layer comprises polycrystalline, microcrystalline, nanocrystalline, ultrananocrystalline or monocrystalline diamond crystals.

4. Implant according to any one of claims 1 to 3, **characterized in that** the carbon-containing layer has diamond crystals with a crystallite size of less than 500 nm.

5. Implant according to claim 4, **characterized in that** the carbon-containing layer has diamond crystals with a crystallite size of 5 to 100 nm.

6. Implant according to claim 5, **characterized in that** the carbon-containing layer has diamond crystals with a crystallite size of 8 to 30 nm.

7. Implant according to any one of claims 1 to 6, **characterized in that** the carbon-containing layer has a layer thickness of 100 nm to 50 µm.

8. Implant according to claim 7, **characterized in that** the carbon-containing layer has a layer thickness of 100 nm to 40 µm.

9. Implant according to claim 8, **characterized in that** the carbon-containing layer has a layer thickness of 1 to 20 µm.

10. Implant according to any one of claims 1 to 9, **characterized in that** the chemically and/or physically modified carbon-containing layer has at least one binding functionality selected from the group consisting of polar, apolar, hydrophobic, hydrophilic, ionic, affine, specific, metal-complexing groups or mixtures thereof.

11. Implant according to any one of claims 1 to 10, **characterized in that** the carbon-containing layer is chemically modified to have hydrogen atoms, halogens, hydroxyl groups, carbonyl groups, aromatic ring systems, sulfur, sulfur derivatives, Grignard compounds, amino groups, epoxides, metals or carbon chains.

12. Implant according to any one of claims 1 to 10, **characterized in that** the carbon-containing layer has a diamond-crystal proportion of at least 90%.

## Revendications

1. Implant comprenant un substrat revêtu au moins partiellement d'une couche contenant du carbone, **caractérisé en ce que** la couche contenant du carbone présente une proportion de cristaux de diamant d'au moins 80 % et est fonctionnalisée au moins en partie avec des facteurs de croissance qui sont fixés directement ou sur au moins un segment de liaison ou un groupe fonctionnel à la couche contenant du carbone.

2. Implant selon la revendication 1, **caractérisé en ce que** la couche contenant du carbone comprend en outre un matériau choisi dans le groupe consistant en des polymères, du carbone amorphe, du DLC (diamond-like-carbon), du graphite, des nanotubes, des nanofils, des nanoparticules, des fullerènes, du pyrocarbone, du carbone vitreux et leurs mélanges.

3. Implant selon la revendication 1 ou 2, **caractérisé en ce que** la couche contenant du carbone comprend des cristaux de diamant polycristallins, microcristallins, nanocristallins, ultrananocristallins ou monocristallins.

4. Implant selon l'une des revendications 1 à 3, **caractérisé en ce que** la couche contenant du carbone présente des cristaux de diamant ayant une dimension de cristaux de moins de 500 nm.

5. Implant selon la revendication 4, **caractérisé en ce que** la couche contenant du carbone présente des cristaux de diamant ayant une dimension de cristaux de 5 à 100 nm.

6. Implant selon la revendication 5, **caractérisé en ce que** la couche contenant du carbone présente des cristaux de diamant ayant une dimension de cristaux de 8 à 30 nm.

7. Implant selon l'une des revendications 1 à 6, **caractérisé en ce que** la couche contenant du carbone présente une épaisseur de couche de 100 nm à 50 µm.

8. Implant selon la revendication 7, **caractérisé en ce que** la couche contenant du carbone présente une épaisseur de couche de 100 nm à 40 µm.

9. Implant selon la revendication 8, **caractérisé en ce que** la couche contenant du carbone présente une épaisseur de couche de 1 à 20 µm.

10. Implant selon l'une des revendications 1 à 9, **caractérisé en ce que** la couche contenant du carbone modifiée chimiquement et/ou physiquement présente au moins une fonctionnalité de liaison choisie dans le groupe consistant en les groupes polaires, apolaires, hydrophobes, hydrophiles, ioniques, affines, spécifiques, formant des complexes métalliques, et leurs mélanges.

11. Implant selon l'une des revendications 1 à 10, **caractérisé en ce que** la couche contenant du carbone présente, par modification chimique, des atomes d'hydrogène, des halogènes, des groupes hydroxyle, des groupes carbonyle, des systèmes cycliques aromatiques, du soufre, des dérivés de soufre, des composés de Grignard, des groupes amino, des époxydes, des métaux ou des chaînes de carbone.

12. Implant selon l'une des revendications 1 à 10, **caractérisé en ce que** la couche contenant du carbone présente une proportion de cristaux de diamant d'au moins 90 %.
